# EUROPEAN PATENT SPECIFICATION

(11) **EP 2 331 152 B1**
(45) Date of publication and mention of the grant of the patent: **21.06.2017**
(21) Application number: 09787118.0
(22) Date of filing: 07.09.2009
(51) Int. Cl.: A61L 27/34, A61L 27/50, A61F 2/00, A61F 2/12

(54) **COATED MEDICAL DEVICE AND METHOD OF COATING A MEDICAL DEVICE TO REDUCE, FIBROSIS AND CAPSULE FORMATION**
BESCHICHTETE MEDIZINISCHE VORRICHTUNG UND BESCHICHTUNGSVERFAHREN FÜR MEDIZINISCHE VORRICHTUNG
DISPOSITIF MÉDICAL REVÊTU ET PROCÉDÉ DE REVÊTEMENT D'UN DISPOSITIF MÉDICAL

(30) Priority: 05.09.2008 EP 08163816
(43) Date of publication of application: 15.06.2011
(73) Proprietor: Ecole Polytechnique Fédérale de Lausanne (EPFL), 1015 Lausanne (CH)
(72) Inventor: MAJD, Hicham, CH-1020 Renens (CH); PIETRAMAGGIORI, Giorgio, CH-1010 Lausanne (CH); HINZ, Boris, Toronto, ON M6P2B7 (CA)
(74) Representative: HGF Limited
(86) International application number: PCT/IB2009/053897
(87) International publication number: WO 2010/026557

(56) References cited:
- WO-A-01/58502
- WO-A-01/81552
- WO-A-03/002710
- WO-A-03/062920
- WO-A-2007/099518
- FALCONNET D ET AL: "Surface engineering approaches to micropattern surfaces for cell-based assays" BIOMATERIALS, ELSEVIER SCIENCE PUBLISHERS BV., BARKING, GB LNKD- DOI:10.1016/J.BIOMATERIALS.2005.12.024, vol. 27, no. 16, 1 June 2006 (2006-06-01), pages 3044-3063, XP025097248 ISSN: 0142-9612 [retrieved on 2006-06-01]
- PARKER J A T C ET AL: "Soft tissue response to microtextured silicone and poly-l-lactic acid implants: fibronectin pre-coating vs. radio-frequency glow discharge treatment" BIOMATERIALS, ELSEVIER SCIENCE PUBLISHERS BV., BARKING, GB, vol. 23, no. 17, 1 September 2002 (2002-09-01), pages 3545-3553, XP004361315 ISSN: 0142-9612

## Description

### FIELD OF THE INVENTION

The present invention relates to a coating technology modulating cell adhesion on the surface of medical devices. The coating prevents capsule formation and decreases fibrosis induced by foreign bodies such as medical devices.

### BACKGROUND OF THE INVENTION

Medical devices in contact with living cells and tissues induce a foreign body reaction due to their physical and chemical properties. This process results in a capsule formation around the implant that often contracts and results in malfunctioning of the device and clinically relevant complications such as pain and dysmorphism of the patient that ultimately require additional surgical operations.

The reaction of cells to a foreign material such as for example a silicone breast and aesthetic implant, a gastric band, a dental implant, an orthopedic implant, heart valve, etc., which cannot be phagocytated, enzymatically digested or otherwise eliminated, is the formation of fibrotic capsule around it.

It has been reported by Prantl L., and al. (Clinical and morphological conditions in capsular contracture formed around silicone breast implants, Plast Reconstr Surg 2007, 120:275-284) that in up to 20-30% of implanted prosthesis develop capsule formation. The capsule formation is excessive and leads to clinically relevant, painful contractures around the device. Patients with excessive capsular formation need to receive a second operation to remove the capsule around the device which considerable increases in the morbidity and costs. This process significantly shortens the lifespan of the device.

This capsule is the result of a chronic inflammatory process around the material that resolves only after the formation of the capsule itself. Myofibroblasts play a main role in capsule formation and contraction in healing tissues. The capsule itself is deposited and contracted by myofibroblasts. In the wound healing process, these highly active cells proliferate and growth to occupy tissue defects and replaces them with a scar. Around the implant, myofibroblast deposit collagen fibers around the foreign material and eventually contract it. This reaction produces capsular contraction.

The problem of capsule formation has also been discussed in patent literature. In US 4,772,285 a collagen coated soft tissue prosthesis is described for reducing capsule formation. While this patent proposes a strategy to decrease capsule formation, no mechanism is provided to effect myofibroblast formation. Further the clinical evidence is lacking. US 4,955,907; 4,731,081; 5,571,183; 5,207,709; 5,354,338; 4,428,082 and 4,298,998 also propose solutions to avoid or diminish capsule formation.

All these documents have several common denominators which have the potential of making them unsuitable for resolving this problem in human beings.

For example US 4,298,998 disclose causing a capsule to form at a predetermined, controlled distance from the surface of the implant, thus resulting in the same capsule but at a different location. The end result clinically appears to be a hard capsule for the patient and not resolving the problem.

Similarly, the implant described in US 5,207,709 includes a plurality of fine projections extending from the outer surface arrayed in a basket weave-like, herringbone-like, or other suitable pattern to create a sinuous path for collagen formation around the implanted device. It appears that this implant actually creates or invites collagen formation again in another location around the implant and again therefore is not resolving the problem.

Still other patents relate to the implant being surrounded by a medical grade elastomer or as described in US 4,944,749 a viscous gel coating with the membranes constructed of a suitable material such as medical grade silicone rubber, which does not react with human tissue. The outer membrane contains an amount of viscous gel, for example a silicone rubber gel of medical grade silicone.

It appears in the end that this patent still has a silicone tissue interface that has accounted for problems.

US 4,610,690 is directed to an implant with a lubricious layer of an acrylamide polymer radiation bonded to at least one wall surface of a silicone shell or bag. Potential long-term effects in human beings of an acrylamide polymer interface are not discussed.

All these aforementioned patents continue to have unnatural chemicals as interface with human tissue, which is exactly what patients do not want in their body and what usually causes problems.

In US 4,995,882 an organic fill solution is proposed to solve the problem. The implant is proposed the use a triglyceride fill substance such as peanut oil or sunflower seed oil as a filling substance. It has been reported by Parker J.A.T.C. et al. (Soft tissue response to microtextured silicone and poly-L-lactic acid implants: fibronectin pre-coating vs. radio-frequency glow discharge treatment, Biomaterials 23, 2002. 3545-3553) that silicone and poly-L-lactic acid implants which are patterned with microgrooves 1 micron deep and 10 microns wide which are coated with a layer of fibronectin, did not significantly improve tissue reaction around the implants. Although some implants of this kind were implanted in Europe, they were never authorized for implantation in the United States and were subsequently taken off the market worldwide because of various problems.

Although many strategies have been developed to reduce capsule formation, from modifications of the surface of the implants to chemical coatings, only mixed results have been reported and, with the increase in the demand for implantable devices, the clinical problem is on the rise.

### SUMMARY OF THE INVENTION

It is therefore an object of the invention to provide a coating of a medical device and a method to produce it with which the fibrosis and capsule contraction can be further diminished or even avoided.

This can be achieved by a medical device wherein a surface of the medical device is coated with cell-adhesive proteins deposited in a matrix of islets as described in claim 1. This coated surface, which is supposed to come in contact with living cells and tissues to modulate cell adhesion is composed from two specific regions:
1. Area (islets) where cell (tissue) can specifically attach (coated part), and
2. Other region where cell (tissue) cannot specifically attach (non coated part).

This invention relates in general to a method and device for guiding cellular adhesion on medical devices. The invention relates to a method comprising protein islets to coat devices (implantable and external) in contact with any cell and tissue of the body.

Specifically, the invention contemplates the use of such technique in combination with devices implanted or externally applied to the body.

This proposal outlines a novel strategy based on islands of proteins to decrease the formation of fibrosis reaction and contraction around medical devices.

According to a preferred embodiment of the invention the medical device comprises silicone as a material of the medical device.

Other preferred embodiments include the use of plastic, metal and/or collagen as a material of the medical device.

According to one embodiment of the invention the medical device according to the invention comprises islets of proteins with uniform geometric shapes.

Another possibility would be the use of different geometric shapes.

Generally, to reduce the myofibroblast differentiation and fibrosis, the area of the single islets is less than 12 µm².
The topography of islets that best reduces fibrosis is composed of single islets, wherein the islets have preferably a length that is <6 µm, a width that is <2 µm and distance between them that is <6 µm

In a preferred embodiment of the invention the medical device further comprises an additional substrate to facilitate the transfer of the islets of proteins.

The substrate which is preferably applied to the medical device before coating same with proteins could be silicone, plastic, bio resistant materials, etc..

Further the invention describes a method of coating a medical device, wherein the medical device is coated with cell-adhesive proteins in form of single islets.

As already mentioned it could be in some embodiments of advantage if the medical device is coated by a substrate before being coated by the proteins.

According to one embodiment of the invention the proteins are applied to the medical device by a stencil or mask (a template with holes of the size and distribution of the islets).

For applying the proteins according to another embodiment the stencil or mask could be brought in contact with the surface of the medical device and the proteins are transferred to the surface through holes of the stencil.

The fixing of the proteins to the medical device could be done by every possibility known from the art. As an example, it should be mentioned covalent binding, electro deposition or precipitation of proteins on the medical device and/or the substrate.

The medical device as described can be used for example for breast implants, tissue expanders, inflatable bumps, implants, transplants, prosthesis, insulin pumps, drug delivery systems, cardiovascular devices, skin substitutes, wound dressings and/or tubes.

### DETAILED DESCRIPTION OF THE INVENTION

These and other objects, features, aspects and advantages of the present invention will become more apparent from the following detailed description of the present invention when taken in conjunction with the accompanying drawings.
Figure 1 shows a medical device before and after being coated according to one embodiment of the invention; and
Figure 2 shows examples of islet shapes according to a preferred embodiment of the invention.

The invention describes a new method of micro-deposition of cell-adhesive proteins and molecules onto the surface of medical devices, (such as, but not limited to silicone), Titanium, Plastic, Polyurethane and generally all materials used for medical devices and implants.

The micro-deposited islets of proteins guide cell adhesion on medical devices with the objective to reduce the fibrotic reaction of the cells and tissues in contact with the device.

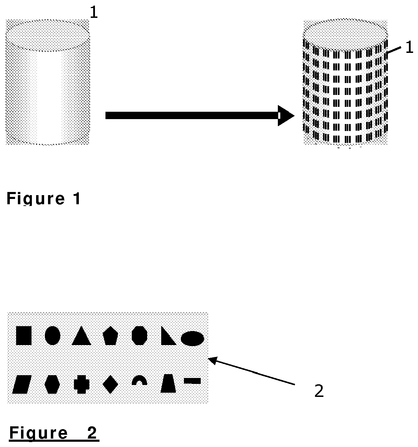

As shown in Figure 1 according to an embodiment of the invention a microperforated stencil or mask is used to transfer proteins to coat a medical device. 1. Deposition of the proteins and molecules is in the form of specific islets. 2. Protein islets include any possible geometrical shape and spatial organization and individual area. Protein islets (2) are deposited to modulate cell and tissue adhesion to medical devices (1).

The distance between the islets may vary from 1 to 50 µm. The area of the single islets being less than 12 µm².

The distribution of protein islets according to a preferred embodiment of the invention to best reduce fibrosis includes single islets, wherein the islets have a length that is <6 µm, a width that is <2 µm and distance between them that is <6 µm.

In another embodiment, deposited islets could have any geometrical shape. Examples to which the invention is not limited are illustrated in Figure 2.

The mask or a stencil to transfer the islets is micro fabricated using technology as for example photolithography, dry and wet etching, laser cutting.

As type of mask or stencil should be mentioned soft stencils made from silicone and flexible polymers and hard stencils from silicon, hard polymer and metal.

To deposit proteins onto medical device as for example an implant surface, the stencil is first brought in conformal contact with the surface and then the proteins are transferred on the surface through the micro-holes of the stencil by covalent binding using 3-aminopropyltriethoxysilane (APTES) and glutaraldehyde or formaldehyde. After the crosslinking of proteins, the stencil is removed and the pattern remains on the implant surface.

Another possible method is electro deposition or precipitation of proteins on implant or medical device surface through the micro-holes of stencil or mask. These possibilities are just examples and should not limit the invention to this approach of deposition.

Our results in vitro and in vivo illustrate the importance of the size of the islets in reducing fibrosis and contraction around implants proving the relevance of this invention. We found that the main mechanism responsible for limiting the differentiation of human dermal fibroblasts in myofibroblasts (the main cell responsible for fibrosis and contraction) is the area of adhesion.

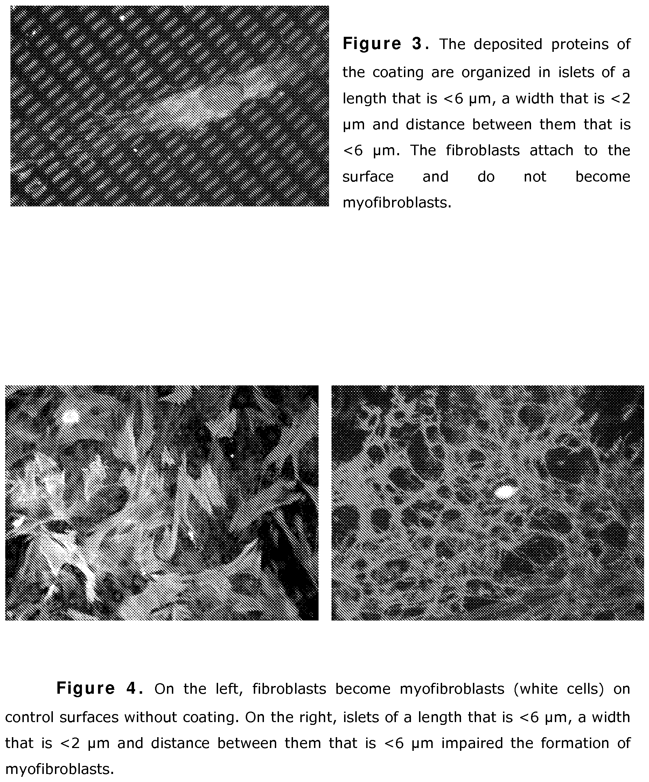

In our experiments, the islets represent areas of adhesion (focal adhesions) for the cells and dimensions of the islets of a length that is <6 µm, a width that is <2 µm and distance between them that is <6 µm impaired the formation of myofibroblasts (Figure 3).

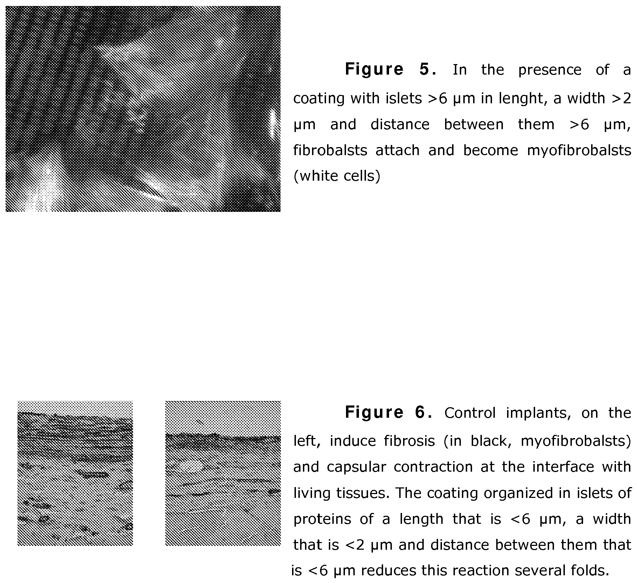

The specific islet size and distribution with a length <6 µm, a width <2 µm and distance between them <6 µm does not allow fibroblasts to exert forces on the surface where they attach and become myofibroblasts (the main cell responsible for fibrosis and contraction). In vitro, we showed that this specific size and distribution of proteins reduced 10-fold the differentiation of human dermal fibroblasts to myofibroblasts compared to devices coated with other size and distribution of proteins (Figure 4). The deposition of islets of higher size allowed the differentiation of fibroblasts in myofibroblasts. These latter cells were seen in high percentage (up to 20%) when the surface was coated with larger islets than the specific ones provided by this invention (Figure 5).

In vivo, silicone pads (1x1 cm) covalently coated with a stencil or mask, as described in the methods, on both side with protein islets with a length <6 µm, a width <2 µm and distance between them <6 µm, were inserted in subcutaneous pockets, in female Wistar rats (250-350 g). On the scapular region of the dorsum of these animals, 4 coated silicone pads were placed. Each animal received four implants: two implants coated with islets with a length <6 µm, a width <2 µm and distance between them <6 µm; and two non coated silicone implants, alternating the location on successive animals. Results at 6 months show a 3-fold decrease in capsule formation around implants coated with islets of proteins with a length <6 µm, a width <2 µm and distance between them <6 µm (Figure 6).

In vitro and in vivo results show that the transformation of fibroblasts into myofibroblasts is crucial in the development of fibrosis and capsule contraction around medical implants and underline the importance of this invention specifically limiting this event.

The optimized protein islets deposition with e.g. a length <6 µm, a width <2 µm and distance between them <6 µm can be applied to any medical device such as, but not limited to:
- Silicone breast implants
- Tissue expanders and inflatable pumps
- Bone, and cartilage and orthopaedic implants
- Tendon, nerve, and ligament transplants, substitutes and implants
- Implantable pumps, such as insuline pumps and drug delivery systems
- Cardiovascular devices, such as pace makers, vascular prosthesis, heart valves, vascular stents
- Skin substitutes and cell scaffolds
- Wound dressings, including dressings and wound interfaces connected to a vacuum (negative pressure dressings)
- Acoustic waves (such as shockwave therapy)
- Ear, throat, nose and eye implants
- Brain, central and peripheral nervous system implants and prosthesis
- Tubes, connecting tubes, drainage tube systems

## Claims

1. A medical device comprising a coating, wherein the coating comprises cell-adhesive proteins having the ability to reduce fibrous reaction, which proteins are composed of collagen or composed of fibronectin or vitronectin or combinations of fibronectin and vitronectin, said coating being in the form of separate islets having an individual area which is less than 12 µm² and wherein the distance between the islets is between 1 µm and 50 µm.

2. The medical device according to claim 1 wherein the distance between the islets is less than 6 µm.

3. The medical device according to one of the previous claims wherein the islets have a uniform geometrical shape.

4. The medical device according to one of the previous claims 1 to 3 wherein the islets have a non-uniform geometrical shape.

5. The medical device according to one of the previous claims wherein the islets have a length that is less than 6 µm, a width that is less than 2 µm and a distance between islets that is less than 6 µm.

6. The medical device according to any one of claims 1 to 5 comprising an additional substrate, which is adapted to facilitate the transfer of the islets of proteins.

7. A process for fixing a coating as defined in one of the previous claims on a medical device, said process comprising a step during which the proteins are applied/transferred by a stencil.

8. The process according to claim 7 wherein said the stencil is brought in contact with the surface of the medical device and the proteins are fixed to the surface through holes in the stencil.

9. A stencil or mask to be used with the process of claim 7 or 8, said stencil or mask optionally being obtained by photolithography, dry or wet etching or laser cutting, wherein the stencil or mask has holes of a size and distribution to produce separate islets having an individual area which is less than 12 µm² and wherein the distance between the islets is between 1 µm and 50 µm.

10. The stencil according to claim 9 being soft and being made from silicone or a flexible polymer.

11. The stencil according to claim 9 being hard and being made from silicon, a hard polymer or metal.

## Patentansprüche

1. Medizinische Vorrichtung, die eine Beschichtung umfasst, wobei die Beschichtung Zelladhäsionsproteine mit der Fähigkeit zum Verringern einer fibrotischen Reaktion umfasst, wobei diese Proteine sich aus Kollagen zusammensetzen oder aus Fibronektin oder Vitronektin oder Kombinationen von Fibronektin und Vitronektin zusammensetzen, wobei die Beschichtung in der Form von getrennten Inseln mit einem Einzelbereich ist, der weniger als 12 µm² beträgt, und wobei der Abstand zwischen den Inseln zwischen 1 µm und 50 µm liegt.

2. Medizinische Vorrichtung nach Anspruch 1, wobei der Abstand zwischen den Inseln weniger als 6 µm beträgt.

3. Medizinische Vorrichtung nach einem der vorhergehenden Ansprüche, wobei die Inseln eine gleichmäßige geometrische Form aufweisen.

4. Medizinische Vorrichtung nach einem der vorhergehenden Ansprüche 1 bis 3, wobei die Inseln eine ungleichmäßige geometrische Form aufweisen.

5. Medizinische Vorrichtung nach einem der vorhergehenden Ansprüche, wobei die Inseln eine Länge, die weniger als 6 µm beträgt, eine Breite, die weniger als 2 µm beträgt, und einen Abstand zwischen Inseln, der weniger als 6 µm beträgt, aufweisen.

6. Medizinische Vorrichtung nach einem der Ansprüche 1 bis 5, die ein zusätzliches Substrat umfasst, das dazu eingerichtet ist, die Übertragung der Inseln von Proteinen zu erleichtern.

7. Verfahren zum Fixieren einer wie in einem der vorhergehenden Ansprüche definierten Beschichtung auf einer medizinischen Vorrichtung, wobei das Verfahren einen Schritt umfasst, während dem die Proteine durch eine Schablone aufgebracht/übertragen werden.

8. Verfahren nach Anspruch 7, wobei die Schablone in Kontakt mit der Oberfläche der medizinischen Vorrichtung gebracht wird und die Proteine durch Löcher in der Schablone auf der Oberfläche fixiert werden.

9. Schablone oder Maske, die mit dem Verfahren nach Anspruch 7 oder 8 verwendet werden soll, wobei die Schablone oder Maske gegebenenfalls durch Photolithographie, Trocken- oder Nassätzen oder Laserschneiden erhalten wird, wobei die Schablone oder Maske Löcher mit einer Größe und einer Verteilung aufweist, um getrennte Inseln mit einem Einzelbereich, der weniger als 12 µm² beträgt, zu produzieren, und wobei der Abstand zwischen den Inseln zwischen 1 µm und 50 µm liegt.

10. Schablone nach Anspruch 9, die weich ist und aus Silikon oder einem flexiblen Polymer hergestellt ist.

11. Schablone nach Anspruch 9, die hart ist und aus Silizium, einem harten Polymer oder Metall hergestellt ist.

## Revendications

1. Dispositif médical comprenant un revêtement, dans lequel le revêtement comprend des protéines d'adhésion cellulaire ayant la capacité d'atténuer une réaction fibreuse, lesdites protéines étant composées de collagène ou composées de fibronectine ou de vitronectine ou de combinaisons de fibronectine et de vitronectine, ledit revêtement se présentant sous la forme d'îlots distincts dont la surface individuelle est inférieure à 12 µm² et dans lequel la distance séparant les îlots est comprise entre 1 µm et 50 µm.

2. Dispositif médical selon la revendication 1, dans lequel la distance qui sépare les îlots est inférieure à 6 µm.

3. Dispositif médical selon l'une quelconque des revendications précédentes, dans lequel les îlots ont une forme géométrique uniforme.

4. Dispositif médical selon l'une quelconque des revendications précédentes 1 à 3, dans lequel les îlots ont une forme géométrique non uniforme.

5. Dispositif médical selon l'une quelconque des revendications précédentes, dans lequel les îlots ont une longueur inférieure à 6 µm, une largeur inférieure à 2 µm et sont séparés par une distance inférieure à 6 µm.

6. Dispositif médical selon l'une quelconque des revendications 1 à 5, comprenant un substrat supplémentaire, ledit substrat étant conçu pour faciliter le transfert des îlots de protéines.

7. Procédé de fixation d'un revêtement tel que défini dans l'une des revendications précédentes à un dispositif médical, ledit procédé comprenant une étape durant laquelle les protéines sont appliquées/transférées par un stencil.

8. Procédé selon la revendication 7, dans lequel ledit stencil est amené en contact avec la surface du dispositif médical et les protéines se fixent à la surface à travers des trous ménagés dans le stencil.

9. Stencil ou masque à utiliser avec le procédé selon la revendication 7 ou 8, ledit stencil ou masque étant éventuellement obtenu par photolithographie, par attaque sèche ou humide ou par découpe au laser, dans lequel le stencil ou masque comporte des trous ayant une taille et une répartition permettant de produire des îlots distincts dont la surface individuelle est inférieure à 12 µm² et dans lequel la distance séparant les îlots est comprise entre 1 µm et 50 µm.

10. Stencil selon la revendication 9, dans lequel ledit stencil est souple et est réalisé en silicone ou en un polymère souple.

11. Stencil selon la revendication 9, dans lequel ledit stencil est dur et est réalisé en silicone, en un polymère dur ou en métal.
